# FASCICULE DE BREVET EUROPEEN

(11) **EP 0 718 271 B1**
(45) Date de publication et mention de la délivrance du brevet: **19.08.1998**
(21) Numéro de dépôt: 95402833.8
(22) Date de dépôt: 15.12.1995
(51) Int. Cl.: C07C 67/04, C07C 69/013, C07C 69/54

(54) **Procédé de préparation de (méth)acrylate d'isobornyle**
Verfahren zur Herstellung von Isobornyl (Meth)acrylat
Process for the preparation of isobornyle (meth)acrylate

(30) Priorité: 22.12.1994 FR 9415473
(43) Date de publication de la demande: 26.06.1996
(73) Titulaire: ELF ATOCHEM S.A., 92800 Puteaux, Hauts-de-Seine (FR)
(72) Inventeur: Paul, Jean-Michel, F-57070 Metz (FR); Desire Gérard, F-62300 Lens (FR)
(74) Mandataire: Rieux, Michel

(56) Documents cités:
- DATABASE WPI Week 8318 Derwent Publications Ltd., London, GB; AN 83-42349 & JP-A-58 049 337 (YASUHARA YUSHI KOGY) , 23 Mars 1983
- DATABASE WPI Week 7423 Derwent Publications Ltd., London, GB; AN 74-42542 & JP-A-49 013 158 (YOSHITOMI PHARM IND LTD) , 5 Février 1974
- DATABASE WPI Week 7011 Derwent Publications Ltd., London, GB; AN 70-17979 & DD-A-69 586 (KLOSE W.)

## Description

La présente invention est relative à un procédé de préparation du méthacrylate d'isobornyle et de l'acrylate d'isobornyle de formule 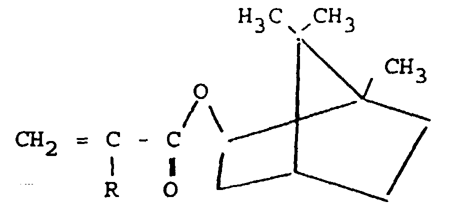 dans laquelle R est un atome d'hydrogène ou un radical méthyle.

Le brevet des Etats-Unis d'Amérique 3.087.962, propose un procédé de préparation de (méth)acrylate d'isobornyle par réaction de l'acide (méth)acrylique sur le camphène de formule 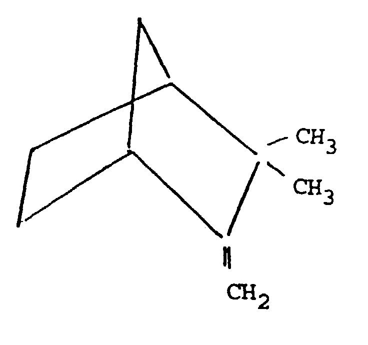 en notant qu'il se produit alors un réarrangement. Le procédé est effectué en présence d'un catalyseur acide fort, tel que l'acide sulfurique ou un acide de Lewis, tel que le trifluorure de bore. Ce procédé ne peut pas être utilisé à l'échelle industrielle en raison de son faible rendement, de la corrosion des réacteurs provoquée par le trifluorure de bore et de la nécessité d'avoir à séparer le catalyseur, ce qui est compliqué.

Pour y remédier, la demande de brevet japonais publiée sous le N°58 049 337 propose un procédé de préparation du (méth)acrylate d'isobornyle du même type que celui rappelé ci-dessus, mais dans lequel la réaction est catalysée par une résine cationique forte sulfonique. La réaction est effectuée en discontinu dans un réacteur agité mécaniquement ou par passage direct des réactifs sur une cartouche remplie de résine. Dans le premier mode de réalisation, les résines peuvent se casser au contact des pales de l'agitateur et il faut une étape de filtration des résines avant la phase de distillation du brut réactionnel. Dans le second mode de réalisation, les vitesses spatiales sont faibles et les temps de séjour sur la résine sont grands. Il s'ensuit une mauvaise élimination de la chaleur engendrée par la réaction, ce qui peut provoquer une grande élévation de la température au sein de la charge de résine avec les dangers que cela comporte de former des sous-produits lourds (de type oligomères) qui peuvent se recraquer au moment de la distillation, ou même dans des cas extrêmes de provoquer une polymérisation de la masse réactionnelle.

L'invention pallie les inconvénients mentionnés ci-dessus par un procédé de préparation de (méth)acrylate d'isobornyle qui simplifie le protocole opératoire en supprimant la filtration du brut réactionnel pour séparer la résine avant distillation, qui permet de faire débuter la phase de distillation immédiatement à la suite de la réaction, qui fait subir moins de contraintes mécaniques aux résines, et qui surtout donne une meilleure qualité du brut réactionnel avec moins de sous-produits lourds et des bilans réactionnels améliorés, tout en ayant, par rapport au procédé avec percolation des réactifs sur une cartouche de résine, une vitesse spatiale plus grande et des temps de séjour moindres sur la résine (de 1 à 2 minutes avec la technique suivant l'invention, alors que dans le document antérieur, cette durée de séjour était de 60 minutes). Les calories sont ainsi plus faciles à éliminer et le moindre danger d'élévation excessive de la température diminue la quantité de sous-produits lourds formés et amoindrit le danger de polymérisation.

Le procédé suivant l'invention consiste à brasser l'acide (méth)acrylique et le camphène dans une cuve agitée pour obtenir un mélange, et à mettre le mélange en contact avec le catalyseur dans une cartouche distincte de la cuve de brassage.

On ne pouvait s'attendre à ce que cette technique, dénommée également, dans le présent mémoire, "technique de la boucle brassée", donne pour la réaction très particulière du camphène et de l'acide (méth)acrylique impliquant un réarrangement, de meilleurs rendement et sélectivité, et moins de sous-produits lourds, tout en diminuant le danger d'une polymérisation. On ne pouvait non plus s'attendre à obtenir ces bons résultats avec une grande vitesse spatiale et une durée de séjour écourtée sur la résine. Le catalyseur solide acide peut être un acide de Broensted ou de Lewis, ou une résine échangeuse d'ion cationique, telle qu'une résine cationique forte macroréticulée à groupements acide sulfonique (cf. Friedrich Hellferich, "Ion Exchange", Mc Graw Hill, N.Y., 1962, p.79 à 88). Les résines acide sulfonique ont de préférence une valeur de pKa inférieure à 1 environ.

La durée de contact du mélange avec la résine exprimée par le rapport du volume de résine gonflée dans le milieu réactionnel au débit de circulation du mélange est de préférence comprise entre 0,1 et 10 minutes, et de préférence entre 1 et 3 minutes.

Le rapport molaire de l'acide (méth)acrylique au camphène peut être compris entre 4/1 et 1/4, et de préférence entre 2/1 et 1/2.

La température de réaction est comprise entre 10 à 60°C, et de préférence entre 30 et 40°C pour le méthacrylate d'isobornyle, et entre 10 et 50°C, et de préférence entre 10 et 30°C pour l'acrylate d'isobornyle.

L'utilisation de solvants bien connus, tels que le cyclohexane, l'hexane, le toluène, est possible.

Il est recommandé d'utiliser des inhibiteurs, tels que l'hydroquinone, l'éther monométhylique de l'hydroquinone, des phénols à fort encombrement stérique, la phénothiazine, pour s'opposer à la polymérisation.

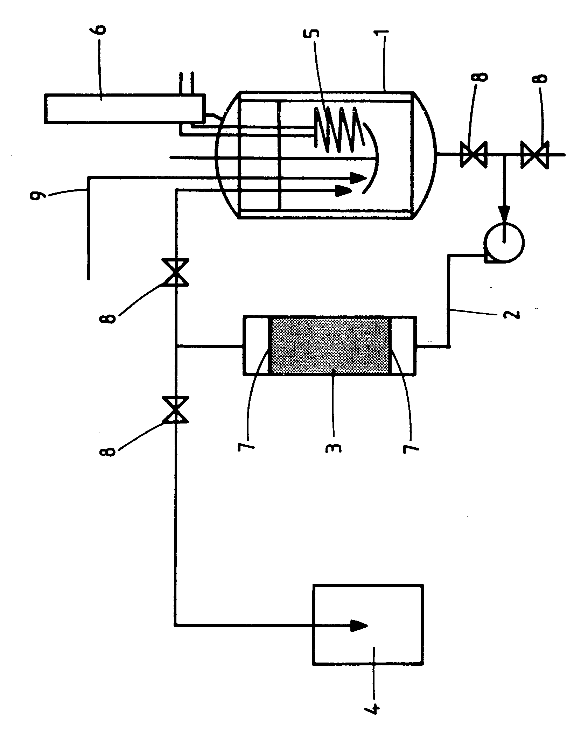

La figure unique du dessin annexé illustre une installation permettant la mise en oeuvre du procédé suivant l'invention.

L'installation se compose :
- d'une cuve de brassage (1) chauffée par double enveloppe et refroidie par un serpentin (5) intérieur. La cuve est surmontée d'un condenseur (6),
- d'une boucle de recirculation (2) sur laquelle est montée une cartouche de résine (3).

La résine est disposée entre deux grilles supports (7).

Des jeux de vannes (8) permettent
- de vidanger la cuve (1) et la boucle de recirculation (2),
- de diriger le flux recirculé vers une capacité intermédiaire (4) lors de la première utilisation de la résine ou en marche normale vers la cuve de brassage (1).

Les réactifs peuvent être introduits directement dans la cuve de brassage (1) par un conduit (9) ou sur la boucle de recirculation (2).

Les exemples suivants illustrent l'invention.

La réalisation pratique de la présente invention s'effectue selon le protocole opératoire ci-après.

Elle met en jeu de l'acide acrylique ou méthacrylique et du camphène pur ou un mélange camphène/tricyclène.

Les réactifs acide acrylique ou acide méthacrylique et camphène (rapport molaire acide/camphène = 1,05/1) sont introduits en tout ou partie au départ dans la cuve de brassage, éventuellement refroidis, puis mis en recirculation forcée à l'aide d'une pompe sur la cartouche de résine.

Le mélange réactionnel est à la sortie de la cartouche (3), renvoyé à la cuve de brassage (1).

La régulation de température (40°C pour le méthacrylate d'isobornyle, 25 à 30°C pour l'acrylate d'isobornyle) se fait par la double enveloppe de chauffage et par le serpentin de refroidissement.

Lorsque tous les réactifs n'ont pas été introduits à la charge, l'ajout du complément de réactifs s'effectue en coulant un mélange acide/camphène pendant 1 à 2 heures, en régulant la température du milieu dans la cuve de brassage.

Lorsque tous les réactifs ont été introduits, on prolonge le temps de mise en réaction de 3 à 5 heures à la température de consigne.

Le brut réactionnel est ensuite distillé sous pression réduite, et les fractions intermédiaires de distillation sont recyclées à l'opération suivante.

La même cartouche de résine est réutilisée plusieurs fois de suite.

### Exemple 1 :

226 g d'acide méthacrylique stabilisé à 250 ppm d'éther méthylique de l'hydroquinone (EMHQ), 340 g de camphène et 0,1 g de phénothiazine sont introduits dans la cuve de brassage (1).

Le mélange est ensuite mis en recirculation sur une cartouche contenant 63 g de résine sèche Amberlyst 15, à un débit de recirculation de 4 litres par heure.

La résine Amberlyst 15 gonfle dans le milieu réactionnel. Le volume de résine humide gonflée est de 75,6 cm³.

Le temps de séjour par passe sur la résine calculé par le rapport volume de résine gonflée/débit de circulation est de 1,8 minute.

La température est régulée à 40°C en sortie résine, la durée de mise en réaction est de 4 heures.

Le brut réactionnel qui renferme 8,9% d'acide méthacrylique, 8,3% de camphène, 81,3% de méthacrylate d'isobornyle, 1,5% de lourds est ensuite distillé sous pression réduite.

Les fractions intermédiaires de distillation riches en acide méthacrylique et camphène sont recyclées à l'opération suivante.

La même charge de résine a été utilisée 6 fois de suite sans baisse de performances.

Le méthacrylate d'isobornyle pur titre 99,8 à 99,9% de pureté.

Les bilans sur l'opération sont les suivants :
- taux de conversion de l'acide méthacrylique : 78%
- taux de conversion du camphène : 86%
- rendement en méthacrylate d'isobornyle(par rapport à l'acide méthacrylique): 75%
- sélectivité(par rapport à l'acide méthacrylique) : 96%

### Exemple 2 :

L'exemple 1 est repris en n'introduisant qu'une partie des réactifs à la charge.

On introduit 201,9 g d'acide méthacrylique stabilisé à 250 ppm d'EMHQ ; 87 g de camphène, 0,15 g de phénothiazine et 0,15 g de Topanol A (2,4 diméthyl 6-tertiobutylphénol) dans la cuve de brassage. Le mélange est mis en recirculation pendant 10 minutes sur la cartouche de résine (63 g de résine sèche Amberlyst 15) à un débit de 4 litres par heure.

Le reste des réactifs est ensuite introduit en une heure sous la forme d'un mélange 20/80 d'acide méthacrylique/camphène (87 g d'acide méthacrylique; 348,2 g de camphène) en régulant la température à 40°C.

Lorsque la coulée est terminée, on prolonge la durée de mise en réaction à 40°C pendant 3 heures et demie.

Le brut, qui renferme 9% d'acide méthacrylique, 8% de camphène, 81% de méthacrylate d'isobornyle, 2% de lourds, est ensuite purifié comme à l'exemple 1.

### Exemple 3 :

L'exemple 1 est repris en remplaçant l'acide méthacrylique par de l'acide acrylique.

234 g d'acide acrylique stabilisé à 200 ppm d'EMHQ, 420 g de camphène, 0,13 g de phénothiazine sont introduits dans la cuve de brassage.

Le mélange est refroidi à 16°C, puis recirculé sur la cartouche de résine (63 g de résine Amberlyst 15 sèche) à un débit de recirculation de 4 litres par heure.

La température de la cuve brassage est régulée à 25-30°C.

La durée de mise en réaction est de 4 heures.

Le brut réactionnel, qui renferme 6% d'acide acrylique, 7% de camphène, 85% d'acrylate d'isobornyle et 2% de lourds, est ensuite distillé sous pression réduite.

Les bilans de l'essai sont les suivants :
- conversion acide acrylique : 83%
- conversion camphène : 89%
- rendement (par rapport à l'acide acrylique) : 79%
- sélectivité (par rapport à l'acide acrylique) : 94%

### Exemple 4 :

On reprend l'exemple 2, en substituant l'acide acrylique à l'acide méthacrylique.

On charge 149,6 g d'acide acrylique stabilisé à 200 ppm d'EMHQ; 84 g de camphène ; 0,13 g de phénothiazine dans la cuve de brassage.

Le mélange réactionnel est refroidi vers 16-18°C, puis mis en recirculation sur la cartouche de résine (63 g Amberlyst 15 sèche).

Après 10 minutes de recirculation à 4 litres par heure, on commence à couler un mélange 20/80 d'acide acrylique/camphène (84 g/336 g) dans la cuve de brassage en régulant la température entre 25 et 30°C.

L'introduction du complément de réactifs s'effectue en une heure.

La durée de mise en réaction est ensuite prolongée de 3 heures à 25-30°C.

Le brut réactionnel, qui renferme 5,5% d'acide acrylique, 6% de camphène, 85% d'acrylate d'isobornyle, 2 à 3% de lourds, est ensuite purifié par distillation sous pression réduite.

Les taux de conversion/rendement/sélectivité sont identiques à ceux de l'essai 3.

### Exemple 5 :

Comparaison procédé discontinu (les réactifs et la résine acide sont mis en contact dans un réacteur agité mécaniquement) et du procédé boucle brassée:

(Les conditions de température, de rapport molaire acide/camphène, de durée globale de mise en réaction en procédé discontinu sont identiques à celles mises en oeuvre en boucle brassée).

| % | | | | |
|---|---|---|---|---|
| | acide méthacrylique | camphène | méthacrylate d'isobornyle | lourds |
| méthacrylate d'isobornyle | | | | |
| discontinu | 9,7 | 9,2 | 78,5 | 2 |
| boucle brassée | 8,9 | 8,3 | 81,3 | 1,5 |

| | acide acrylique | % camphène | acrylate d'isobornyle | lourds |
|---|---|---|---|---|
| acrylate d'isobornyle | | | | |
| discontinu | 6,5 | 6 | 83 | 4,5 |
| boucle brassée | 6 | 6 | 85 | 3 |

L'exemple 5 montre, qu'outre les avantages pratiques conférés par le procédé. en boucle brassée (meilleur contrôle de l'exothermie de la réaction, suppression de l'étape de filtration de la résine), on obtient un brut réactionnel qui renferme moins de sous-produits lourds susceptibles de rétrograder en acide difficile à séparer du méthacrylate d'isobornyle et surtout de l'acrylate d'isobornyle lors de la distillation.

## Revendications

1. Procédé de préparation de (méth)acrylate d'isobornyle de formule : 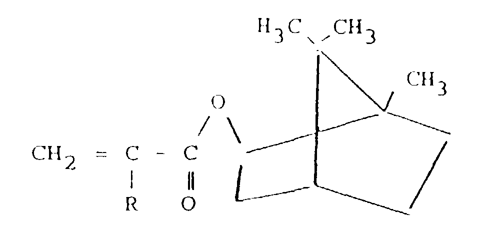 dans laquelle R est un atome d'hydrogène ou un radical méthyle par réaction de l'acide (méth)acrylique de formule: 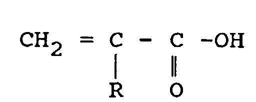 sur le camphène de formule : 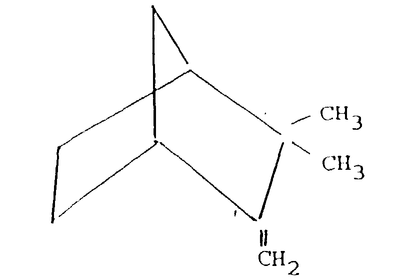 en présence d'un catalyseur solide acide, caractérisé en ce qu'il consiste à brasser l'acide (méth)acrylique et le camphène dans une cuve de brassage pour obtenir un mélange et à mettre le mélange en contact avec le catalyseur dans une cartouche distincte de la cuve de brassage.

2. Procédé selon la revendication 1, caractérisé en ce que le camphène est introduit sous la forme de camphène pur ou sous la forme d'un mélange camphène-tricyclène.

3. Procédé suivant l'une des revendications 1 et 2, caractérisé en ce que la durée de contact du mélange avec la résine, exprimée par le rapport du volume de résine gonflée dans le milieu réactionnel au débit de passage du mélange, est comprise entre 0,1 et 10 minutes, et de préférence entre 1 et 3 minutes.

4. Procédé suivant l'une des revendications 1 à 3, caractérisé en ce que le rapport molaire de l'acide (méth)acrylique au camphène est compris entre 4/1 et 1/4, et de préférence entre 2/1 et 1/2.

5. Procédé suivant l'une des revendications 1 à 4, caractérisé en ce qu'il consiste à mettre le mélange en contact avec le catalyseur à une température comprise entre 10 et 60°C, et de préférence entre 30 et 40°C, pour le (méth)acrylate d'isobornyle, et entre 10 et 50°C, et de préférence entre 10 et 30°C pour l'acrylate d'isobornyle.

6. Procédé selon l'une des revendications 1 à 5, caractérisé par le fait que le catalyseur solide acide est choisi parmi les acides de Brönsted, les acides de Lewis et les résines acides échangeuses d'ions cationiques, telles que les résines cationiques fortes macroréticulées à groupements acide sulfonique.

## Patentansprüche

1. Verfahren zur Herstellung von Isobornyl(meth)acrylat der Formel 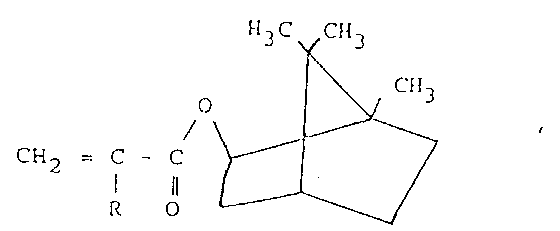 in der R ein Wasserstoffatom oder Methyl bedeutet,
durch Umsetzung von (Meth)acrylsäure der Formel: 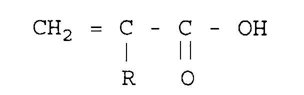 mit dem Camphen der Formel 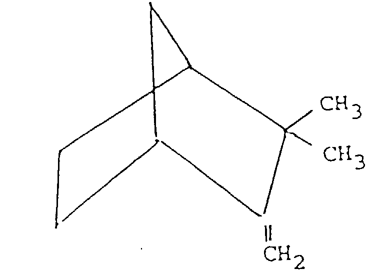 in Gegenwart eines festen sauren Katalysators,
gekennzeichnet durch
Vermischen der (Meth)acrylsäure mit dem Camphen in einem Mischgefäß zu einem Gemisch und Inkontaktbringen des Gemisches mit dem Katalysator in einem vom Mischgefäß verschiedenen Behälter.

2. Verfahren nach Anspruch 1, dadurch gekennzeichnet, daß das Camphen in Form von reinem Camphen oder in Form eines Camphen-Tricyclen-Gemischs eingesetzt wird.

3. Verfahren nach Anspruch 1 oder 2, dadurch gekennzeichnet, daß die Dauer des Kontakts des Gemisches mit dem Harz, ausgedrückt als Verhältnis des Volumens des im Reaktionsmedium gequollenen Harzes zum Durchsatz an Gemisch, im Bereich von 0,1 bis 10 min und vorzugsweise im Bereich von 1 bis 3 min liegt.

4. Verfahren nach einem der Ansprüche 1 bis 3, dadurch gekennzeichnet, daß das Molverhältnis von (Meth)acrylsäure zu Camphen im Bereich von 4/1 bis 1/4 und vorzugsweise im Bereich von 2/1 bis 1/2 liegt.

5. Verfahren nach einem der Ansprüche 1 bis 4, gekennzeichnet durch Inkontaktbringen des Gemisches mit dem Katalysator bei einer Temperatur im Bereich von 10 bis 60 °C und vorzugsweise im Bereich von 30 bis 40 °C im Fall von Isobornylmethacrylat und im Bereich von 10 bis 50 °C und vorzugsweise im Bereich von 10 bis 30 °C im Fall von Isobornylacrylat.

6. Verfahren nach einem der Ansprüche 1 bis 5, dadurch gekennzeichnet, daß der feste saure Katalysator unter Brönsted-Säuren, Lewis-Säuren und sauren Kationenaustauscherharzen, wie makrovernetzten starken Kationenaustauscherharzen mit Sulfonsäuregruppen, ausgewählt wird.

## Claims

1. Process for the preparation of isobornyl (meth)acrylate of formula: 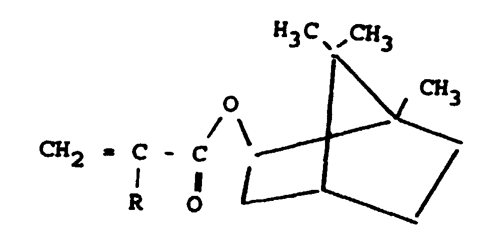 in which R is a hydrogen atom or a methyl radical, by reaction of (meth)acrylic acid, of formula: 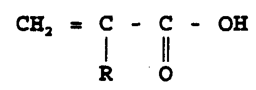 with the camphene of formula: 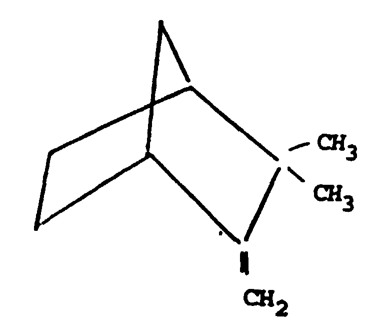 in the presence of a solid acid catalyst, characterized in that it consists in blending (meth)acrylic acid and camphene in a blending tank in order to obtain a mixture and in placing the mixture in contact with the catalyst in a cartridge which is separate from the blending tank.

2. Process according to Claim 1, characterized in that the camphene is introduced in the form of pure camphene or in the form of a camphene-tricyclene mixture.

3. Process according to either of Claims 1 and 2, characterized in that the duration of contact of the mixture with the resin, expressed by the ratio of the volume of swollen resin in the reaction medium to the flow rate of passage of the mixture, is between 0.1 and 10 minutes, and preferably between 1 and 3 minutes.

4. Process according to one of Claims 1 to 3, characterized in that the molar ratio of the (meth)acrylic acid to the camphene is between 4/1 and 1/4, and preferably between 2/1 and 1/2.

5. Process according to one of Claims 1 to 4, characterized in that it consists in placing the mixture in contact with the catalyst at a temperature between 10 and 60°C, and preferably between 30 and 40°C, for isobornyl (meth)acrylate, and between 10 and 50°C, and preferably between 10 and 30°C, for isobornyl acrylate.

6. Process according to one of Claims 1 to 5, characterized in that the solid acid catalyst is chosen from Brönsted acids, Lewis acids and acidic cation-exchange resins, such as macro-crosslinked strong cationic resins containing sulphonic acid groups.
